# FASCICULE DE BREVET EUROPEEN

(11) **EP 0 928 607 B1**
(45) Date de publication et mention de la délivrance du brevet: **02.05.2001**
(21) Numéro de dépôt: 98403146.8
(22) Date de dépôt: 14.12.1998
(51) Int. Cl.: A61K 7/032, A61K 7/48

(54) **Mascara comprenant un mélange de cires dures et de polymère filmogène**
Eine Mischung harter Wachse und filmbildendes Polymer enthaltendes Mascara
Mascara composition having a mixture of hard waxes and a film-forming polymer

(30) Priorité: 31.12.1997 FR 9716806
(43) Date de publication de la demande: 14.07.1999
(73) Titulaire: L'OREAL, 75008 Paris (FR)
(72) Inventeur: Piot, Bertrand, 92250 La Garenne Colombes (FR); Debert, Danièle, 91600 Savigny-sur-Orge (FR); Bodelin-Lecomte, Sophie, 92170 Vanves (FR)
(74) Mandataire: Lhoste, Catherine

(56) Documents cités:
- EP-A- 0 530 084
- EP-A- 0 557 196
- EP-A- 0 655 234
- EP-A- 0 663 202
- GB-A- 2 123 290

## Description

La présenté invention a pour objet l'utilisation d'une composition de mascara, comprenant un mélange de cires et de polymère filmogène pour recourber et épaissir les fibres kératiniques, notamment les cils ou les pointes des cheveux. Elle a aussi pour objet une composition de mascara comprenant un mélange de cires et un polymère filmogène.

Il est d'usage courant de réaliser des compositions de mascara contenant au moins une cire. Toutefois, celle-ci n'est jamais utilisée seule, car le maquillage avec de-telles compositions s'avère très médiocre conduisant à la formation sur les cils d'un film non homogène qui se traduit par la formation de pellicules craquantes, immédiatement après le séchage.

Il est également connu selon les demandes WO 96/36323 et WO 96/33690 d'associer une cire et un polymère filmogène dans une composition de mascara. Toutefois, une telle association ne permet pas de bien recourber les cils, ni d'obtenir un maquillage épais des cils.

Il a aussi été proposé dans les demandes EP-A-557196 et EP-A-639371 des compositions de mascara contenant des microdispersions de cires associées à des polymères filmogènes. Toutefois, de telles compositions ne permettent pas d'obtenir un maquillage épais des cils : de tels mascaras sont donc peu chargeant.

Le brevet GB-A-2 123 290 décrit une composition de mascara comprenant (voir exemple 2):
(i) une émulsion cires-dans-eau de cire de carnauba et de cire de. candelilla, leur mélange étant présent en une teneur d'au moins 10 % en poids par rapport au poids total de la composition; la taille des particules n' est toutefois pas indiquée,
(ii) au moins 0,1 % en poids, par rapport au poids total de la composition, de deux dérivés cellulosiques et de polyméthacrylate de sodium (Darvan 7, Polymer JR 400 et Hydroxyethylcellulose)
et son utilisation pour allonger les cils. L'utilisation pour recourber et épaissir les fibres kératiniques n'est cependant pas décrite.

L'invention a pour but de proposer une-composition cosmétique permettant un recourbement amélioré des cils et l'obtention de cils chargés en maquillage. L'invention a pour but également de proposer une composition conférant un recourbement instantané des cils, durable dans le temps et constituant un maquillage bien toléré par les yeux sensibles.

La demanderesse a maintenant constaté de façon surprenante qu'un tel maquillage des cils pouvait être obtenu en utilisant une association de cires et de polymères filmogènes particuliers.

Ainsi, un objet de l'invention est l'utilisation d'une composition de mascara pour recourber et épaissir les fibres kératiniques, notamment les cils, comprenant
- (i) une émulsion cires-dans-eau d'au moins une cire I, ou un mélange de cires I, ayant une pénétrabilité à l'aiguille allant de 1 à 7,5 et un point de fusion allant de 70 °C à 110 °C, ladite cire I comprenant au moins une cire (appelée(s) par la suite la et/ou Ib) ayant un point de fusion allant de 77 °C à 110 °C, ladite cire I, ou leurs mélanges, étant présent en une teneur d'au moins 10 % en poids par rapport au poids total de la composition, la cire, ou leur mélange, étant sous forme de particules ayant une taille au moins supérieure ou égale à 1 µm,
- (ii) au moins 0,1 % en poids, par rapport au poids total de la composition, d'un système polymérique contenant un polymère filmogène, ledit système polymérique étant apte à former un film produisant à une concentration de 7 % dans l'eau,

Un autre objet de l'invention est une composition de mascara comprenant :
- (i) une émulsion cires-dans-eau d'au moins un mélange de cires I, dites cires dures, ayant une pénétrabilité à l'aiguille allant de 1 à 7,5 et un point de fusion allant de 70 °C à 110 °C, ledit mélange de cires I contenant au moins une première cire (la) ayant un point de fusion supérieur ou égal à 77 °C et inférieur à 83 °C, une deuxième cire (Ib) ayant un point de fusion allant de 83 °C à 110 °C, et une troisième cire (Ic) ayant un point de fusion supérieur ou égal à 70 °C et inférieur à 77 °C, ledit mélange de cires t étant présent en une teneur d'au moins 10 % en poids par rapport au poids total de la composition, ledit mélange de cires I étant sous forme de particules ayant une taille au moins supérieure ou égale à 1 µm,
- (ii) 0,1 % en poids, par rapport au poids total de la composition, d'un système polymérique contenant un polymère filmogène, ledit système polymérique étant apte à former un film produisant à une concentration de 7 % dans l'eau, une rétraction du stratum corneum isolé de plus de 1 % à 30°C sous une humidité relative de 40 %.

Un autre objet de l'invention est un produit de mascara comprenant un réservoir contenant une composition de mascara telle que définie précédemment, et muni d'un système d'application de la composition sur les fibres kératiniques, notamment les cils.

Un autre objet de l'invention est un procédé de maquillage des fibres kératiniques, notamment des cils, consistant à appliquer sur les fibres kératiniques une composition telle que définie précédemment.

Selon l'invention, les cires sont sous forme de particules ayant de préférence une taille supérieure ou égale à 1,5 µm, notamment allant de 1,5 à 10 µm, et mieux allant de 1,5 µm à 3,5 µm.

Par cire dure (ou cire (I)), on entend une cire ayant un point de fusion allant de 70 °C à 110 °C et une pénétrabilité à l'aiguille allant de 1 à 7, 5. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Les cires I utilisées conformément à l'invention peuvent être choisies parmi les cires d'origine animale, les cires d'origine végétale, les cires d'origine minérale, les cires synthétiques et les fractions diverses de cires d'origine naturelle, toutes ces cires présentant les deux caractéristiques (pénétrabilité à l'aiguille, point de fusion) indiquées ci-dessus.

Les cires (I) peuvent être choisies notamment parmi la cire de son de riz, la cire de Carnauba, la cire d'Ouricuri, la cire de Candellila, les cires de Monatan, la cire de canne à sucre, certaines cires de polyéthylène qui répondent aux critères des cires I.

Avantageusement, la composition selon l'invention peut comprendre une quantité du mélange de cires I allant de 10 % à 30 % en poids, par rapport au poids total de la composition, de préférence de 13 % à 25 %, et mieux au moins 15 % et notamment de 15 % à 20 %.

La cire I ou le mélange de cires I peut comprendre au moins une première cire (la) ayant un point de fusion supérieur ou égal à 77 °C et inférieur à 83 °C. Une telle cire peut être par exemple la cire de son de riz.

La cire I ou le mélange de cires I peut comprendre également au moins une deuxième cire (Ib) ayant un point de fusion allant de 83 °C à 110 °C. Cette deuxième cire (Ib) peut être utilisée seule dans le mélange de cires I ou être associée à la cire (la). Une telle cire (Ib) peut être par exemple la cire de Carnauba, la cire d'Ouricuri, les cires de Montan. On utilise de préférence la cire de Carnauba.

La cire 1 ou le mélange de cires I peut comprendre en outre une troisième cire (Ic) ayant un point de fusion supérieur ou égal à 70 °C et inférieur à 77 °C. Une telle cire peut être par exemple la cire de Candelilla.

Pour l'utilisation selon l'invention, la composition de mascara comprend de préférence un mélange de cires I contenant au moins une première cire (la) et au moins une deuxième cire (Ib) telles que définies précédemment.

Ledit mélange de cires 1 peut comprendre de 35 % à 65 % en poids de cire (la), par rapport au poids total dudit mélange de cires I, et de 65 % à 35 % en poids de cire (lb).

De manière préférée, ledit mélange de cires I peut comprendre en plus des première et deuxième cires (la, Ib), une troisième cire (Ic) telle que définie précédemment. Cette troisième cire peut être présente dans la composition en une teneur allant de 5 % à 20 % en poids, par rapport au poids total du mélange de cires I.

Avantageusement, les première, deuxième et troisième cires (la, Ib, lc) peuvent être présentes dans la composition selon un rapport pondéral (poids/poids total de cires I) allant respectivement de :
- cire (la) : 0,35 à 0,5,
- cire (lb) : 0,35 à 0,5,
- cire (lc) : 0,05 à 0,2.

Pour l'utilisation selon l'invention, la composition comprend en plus du mélange de cire I au moins un système polymérique apte à former un film produisant à une concentration de 7 % dans l'eau, une rétraction du stratum corneum isolé de plus de 1 %, à 30°C sous une humidité relative de 40 %, de préférence de plus de 1,2 % et mieux de plus de 1,5 %. Cette rétraction est mesurée au dermomètre, selon la méthode décrite ci-après. De tels polymères confèrent un très bon recourbement aux cils.

Par « système polymérique », on entend soit un polymère seul soit un polymère associé à au moins un autre polymère soit un polymère associé à au moins un agent plastifiant de façon à obtenir les caractéristiques mécaniques recherchées.

Par « apte à former un film », on entend un système polymérique permettant de former un film : lorsqu'on l'étale sur du verre, le système polymérique doit sécher sans se craqueler.

Le polymère filmogène du système polymérique selon l'invention peut être un polymère d'origine naturelle ou un polymère synthétique. Le polymère filmogène peut être notamment un polymère hydrosoluble ou hydrodispersible.

Par polymère d'origine naturelle, on entend les polymères d'origine végétale, d'origine animale.

Comme polymères d'origine végétale, on peut citer notamment les protéines et hydrolysats de protéines, et plus particulièrement les extraits de céréales, de légumineuses et d'oléagineuses, tels que les extraits de blé, de maïs, de seigle, de froment, de sarrasin, de sésame, d'épeautre, de pois, de fève, de lentille, de soja et de lupin.

Comme polymère d'origine animale, on peut utiliser des polymères issus des carapaces d'insectes ou crustacés. On peut citer par exemple la chitine et ses dérivés, notamment le chitosane qui est un dérivé déacétylé de la chitine, ainsi que les dérivés du chitosane tels que l'hydroxypropylchitosane, le dérivé succinylé de chitosane, le lactate de chitosane, le glutamate de chitosane, le succinamide de carboxyméthylchitosane.

Les polymères d'origine naturelle, éventuellement modifiés, peuvent être choisis par exemple parmi la résine shellac, la gomme de sandaraque, les dammars, les élémis, les copals, les dérivés cellulosiques.

Comme polymères d'origine naturelle hydrosolubles appropriés, on peut citer l'hydroxypropyl chitosane vendu sous la dénomination "HPCH powder" par la société ICHIMARU PHARCOS, l'hydrolysat de protéine de blé vendu sous la dénomination "Tritisol" par la société Croda (ayant un poids moléculaire d'environ 250 000 daltons).

Les polymères synthétiques peuvent être de type polycondensat ou de type radicalaire.

Comme polycondensats, on peut citer les polyuréthannes anioniques, cationiques, non ioniques ou amphotères, les polyuréthannes-acryliques, les polyuréthannes-polyvinylpyrrolidones, les polyester-polyuréthannes, les polyéther-polyuréthannes, les polyurée-uréthanes, les polyurées, les sulfopolyesters (qui sont décrits notamments dans les brevets US-A-3734874, US-A-4233196, US-A-4304901) et leurs mélanges.

Comme polymères radicalaires, on peut citer les polymères acryliques, les copolymères acrylique/styrène, les copolymères vinyliques comme les copolymères d'esters vinyliques.

Comme polymère synthétique approprié, on peut citer notamment les dispersions de polyester-polyuréthanne, commercialisées sous les dénominations "Sancure 2060" (polyester-polyuréthanne), "Sancure 815" (polyester-polyuréthanne) ou bien encore les sulfopolyesters à base d'isophtalate/sulfoisophtalate, et plus particulièrement les sulfopolyesters obtenus par condensation de di-éthylèneglycol, cyclohexane di-méthanol, acide isophtalique, acide sulfoisophtalique, notamment ceux commercialisés sous les dénominations AQ55S par la société EASTMAN.

Lorsque le polymère filmogène ne permet pas d'obtenir seul un film ayant les caractéristiques mentionnées précédemment, il est possible d'ajouter un composé dont la fonction est de modifier les propriétés du polymère filmogène pour obtenir le système polymérique souhaité. Aussi, selon un mode de réalisation de la composition selon l'invention, ledit système polymérique peut comprendre au moins un agent auxiliaire de filmification permettant d'obtenir un film ayant les caractéristiques telles que décrites précédemment. L'agent auxiliaire de filmification permet notamment d'obtenir un film permettant d'obtenir un bon recourbement des cils. Dans ce cas, le système polymérique comprend un mélange d'un ou plusieurs polymères filmogènes et d'au moins un agent auxiliaire de filmification.

Un tel agent auxiliaire de filmification peut être choisi parmi tous les composés connus de l'homme du métier comme étant susceptibles de remplir la fonction recherchée, et être notamment choisi parmi les agents plastifiants. En outre, lorsque le système polymérique selon l'invention comprend au moins une dispersion aqueuse de particules de polymère filmogène, l'agent auxiliaire de filmification peut aussi être choisi parmi les agents de coalescence. Cet agent auxiliaire peut être hydrosoluble ou insoluble dans l'eau et peut éventuellement se présenter sous forme de dispersion aqueuse.
En particulier, on peut citer, seuls ou en mélange, les plastifiants ou agents de coalescence usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ayant une température de transition vitreuse faible, inférieure à 25°C, de préférence inférieure à 15°C.

La quantité d'agent auxiliaire de filmification peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables.

La composition peut comprendre de 0,1 % à 10 % en poids, de préférence de 0,3 % à 7 % , de matière sèche de polymères filmogènes, par rapport au poids total de la composition.

Le système polymérique utilisé (polymère(s) ou polymère et plastifiant) selon l'invention peut être notamment présent en une quantité de matière active (M.A.) allant de 0,1 à 15 %, et mieux de 0,3 à 10 % du poids total de la composition.

En outre, la composition pour l'utilisation selon l'invention peut comprendre au moins une cire Il, dite cire molle, ayant un point de fusion supérieur ou égal à 50 °C et inférieur à 70 °C, et une pénétrabilité à l'aiguille supérieure à 7,5, et de préférence inférieure ou égale à 217, mesurée selon les conditions définies précédemment pour les cires I. Cette cire Il permet notamment d'assouplir le maquillage déposé sur les cils.

Ces cires Il peuvent être notamment choisies parmi la cire d'abeilles, les cires de lanoline, les cires de paraffine, les cires de cérésine, les cires microcristallines, les spermaceti, certaines cires de polyéthylène de poids moléculaire tel qu'elles répondent aux critères des cires II, les huiles végétales hydrogénées.

Parmi les huiles végétales hydrogénées, on peut citer les cires de jojoba hydrogénées et les huiles hydrogénées qui sont obtenues par hydrogénation catalytique de corps gras composés de chaîne grasse linéaire ou non en C₈-C₃₂ et qui ont les qualités correspondant à la définition des cires. On peut citer notamment l'huile de tournesol hydrogénée, l'huile de ricin hydrogénée, l'huile de coprah hydrogénée et la lanoline hydrogénée.

Avantageusement, les cires I et les cires Il sont présentes dans la composition selon un rapport pondéral cires I / cire II pouvant aller de 2 à 5, et de préférence de 2,5 à 3,5.

La composition pour l'utilisation selon l'invention peut contenir des agents tensioactifs émulsionnants présents en une proportion allant de 2 à 30 % en poids par rapport au poids total de la composition, et mieux de 5 % à 20 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans les compositions selon l'invention sont :
- parmi les tensioactifs non-ioniques: les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés.
- parmi les tensioactifs anioniques : les acides gras en C16-C30 neutralisés par les amines, l'ammoniaque ou les sels alcalins.

On utilise de préférence des tensioactifs permettant l'obtention d'émulsion huile-dans-eau.

Dans la composition selon l'invention, l'eau peut représenter avantageusement de 30 à 80% en poids du poids total de la composition.

En outre, la composition peut comprendre aux moins un agent épaississant, de préférence de nature hydrophile. Celui-ci peut par exemple être choisi parmi les polymères carboxyvinyliques (carbomer), les copolymères acryliques tels que les copolymères d'acrylates/alkylacrylates, les polyacrylamides, les polysaccharides, les gommes naturelles et les argiles.

La composition peut comprendre avantageusement un tensioactif siliconé ayant un HLB allant de 8 à 16. De préférence, un tel tensioactif est un diméthicone copolyol. Lorsque la composition selon l'invention est déposée sur les cils, le tensioactif siliconé permet une meilleure tenue dans le temps et une meilleure résistance aux sollicitations mécaniques de la composition ainsi déposée.
Les diméthicone copolyols peuvent être choisis parmi les composés de formule générale (I) 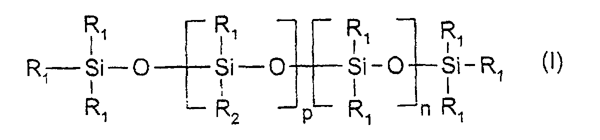 formule dans laquelle :
- R₁, identiques ou différents, représentent un atome d'hydrogène, un radical alkyle linéaire ou ramifié en C₁-C₃₀ ou un radical phényle,
- R₂, identiques ou différents, représentent -(CₓH₂ₓ-(OC₂H₄)ₐ-(OC₃H₆)_{b}-OR₃,
- R₃, identiques ou différents, sont choisis parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 0 à 1000,
- p varie de 1 à 30,
- a varie de 0 à 50,
- b varie de 0 à 50,
- a + b est supérieur ou égal à 1,
- x varie de 1 à 5,
- le poids moléculaire moyen en nombre étant supérieur ou égal à 15000 et de préférence compris entre 25000 et 75000.

De façon préférentielle, on utilise les silicones oxyalkylénées de formule générale (I) qui répondent à au moins une des, et de préférence à toutes les, conditions suivantes :
- R₁ désigne le radical méthyle.
- R₃ représente un atome d'hydrogène, un radical méthyle ou un radical acétyle, et de préférence hydrogène.
- p varie de 8 à 20.
- a est compris entre 5 et 40 et de préférence entre 15 et 30.
- b est compris entre 5 et 40 et de préférence entre 15 et 30.
- x est égal à 2 ou 3.
- n varie de 20 à 600, de préférence de 50 à 500 et encore plus particulièrement de 100 à 300.
De telles silicones sont par exemple décrites dans le brevet US-4,311,695.
Des diméthicones copolyols ont en particulier été présentés par la société DOW CORNING lors du 17ème congrès international de l'I.F.S.C.C. d'octobre 1992 et rapportés dans l'article "Water-soluble dimethicone copolyol waxes for personal care industry" de Linda Madore et al., pages 1 à 3.
Ces diméthicones copolyols sont des polydiméthylsiloxanes (PDMS) comportant une ou plusieurs fonctions éthers, solubles dans l'eau (oxyalkylène, notamment oxyéthylène et/ou oxypropylène).
De tels diméthicones copolyols sont notamment vendus par la société GOLDSCHMIDT sous la dénomination ABIL B8851 ou ABIL 888183. On peut citer aussi les composés KF 351 à 354 et KF 615 A vendus par la société SHIN ETSU ou la DMC 6038 de la société WACKER.
Les dérivés de diméthicones copolyols utilisables peuvent être en particulier les diméthicones copolyols à groupement phosphate, sulfate, chlorure de myristamide propyldiméthylammonium, stéarate, amine, glycomodifié, etc. On peut utiliser comme dérivés de diméthicones copolyols notamment les composés vendus par la société SILTECH sous la dénomination Silphos A100, Siltech amine 65, Silwax WDIS, myristamido silicone quat, ou par la société PHOENIX sous la dénomination Pecosil PS 100.
On peut également utiliser les dérivés vendus par la société WACKER sous la dénomination VP 1661, ou par la société DOW CORNING sous la dénomination 2501 cosmetic wax. On peut encore utiliser les dérivés vendus par la société WACKER sous la dénomination VP 1661, ou par la société DOW CORNING sous la dénomination 2501 cosmetic wax.
Les silicones les plus particulièrement préférées sont par exemple celles vendues par la société DOW CORNING sous la dénomination commerciale Q2-5220 et par la société RHONE POULENC sous la dénomination MIRASIL DMCO.

La composition selon l'invention peut également contenir des ingrédients couramment utilisés en cosmétique, tels que les vitamines, les oligo-éléments, les adoucissants, les séquestrants, les parfums, les huiles, les silicones, les agents de cohésion ainsi que les agents alcalinisants ou acidifiants habituellement utilisés dans le domaine cosmétique, les charges, les pigments, les émollients, habituellement utilisés en quantités comprises entre 1 et 10% ; les conservateurs.

La composition selon l'invention est destiné à un produit de mascara comprenant un réservoir, contenant ladite composition de mascara, et un système d'application de ladite composition sur les fibres kératiniques, notamment les cils. Le réservoir est muni de façon connue d'une ouverture dans laquelle est logée un système d'essorage. Le système d'application comporte une tige munie à une première extrémité d'une brosse et à une deuxième extrémité d'un bouchon destiné à fermer le réservoir. Un tel conditionnement est notamment illustré à la figure 7 de la demande EP-A-611170.

L'invention est illustrée plus en détail dans les exemples suivants.

### Méthode de mesure de rétraction :

Le principe consiste à mesurer avant traitement et après traitement la longueur d'une éprouvette de stratum cornéum isolé et de déterminer le pourcentage de rétraction de l'éprouvette.

On utilise des éprouvettes de 1 cm X 0,4 cm de stratum cornéum d'épaisseur allant de 10 à 20 µm disposées sur l'extensiomètre MTT 610 commercialisé par la société DIASTRON.

L'éprouvette est placée entre 2 mâchoires puis laissée pendant 12 heures dans une atmosphère à 30 °C et 40 % d'humidité relative.

On tracte à la vitesse de 2 mm/minute l'éprouvette d'une longueur comprise entre 5 et 10 % de la longueur initiale pour déterminer la longueur l₁ à partir de laquelle l'éprouvette commence à exercer une force sur les mâchoires et détectée par l'appareil.

On détend ensuite l'éprouvette puis on applique sur le stratum cornéum 2 mg d'une composition aqueuse à 7 % en poids de polymère. Après évaporation totale de la composition, on tracte l'éprouvette dans les mêmes conditions que celles décrites précédemment pour déterminer également la longueur l₂.pour l'éprouvette traitée.

Le pourcentage de rétraction est déterminé par le rapport : 100 X (I₂ - I₁)/I₁.

### Exemple 1 :

On a préparé un mascara ayant la composition suivante :
- cire de son de riz 7 g
- cire de carnauba 7 g
- cire de Candelilla 2,8 g
- stéarate de triéthanolamine 8,4 g
- cire d'abeille 6 g
- hydrolysat de protéine de blé vendu sous la dénomination "Tritisol" par la société CRODA 0,31 g MA
- hydroxyéthyl cellulose 1,5 g
- pigments 8 g
- conservateurs qs
- eau qsp 100 g

Les cires, le tensioactif et les conservateurs ont été fondus et mélangés ensemble à 90 °C. Les pigments ont été dispersés dans le mélange fondu à 90 °C. Le Tritisol et l'hydroxyéthylcellulose ont été dissous dans l'eau à froid. La phase aqueuse a ensuite été chauffée à 90 °C et versée dans le mélange fondu sous agitation, tout en maintenant la température vers 90 °C jusqu'à homogénéité. Puis la composition a été refroidie à température ambiante.

On a ainsi obtenu un mascara se présentant sous la forme d'une dispersion cires-dans-eau dont les particules de cires ont une taille supérieure à 1,5 µm.

En appliquant le mascara sur les cils, on a constaté que ceux-ci présentaient un très bon recourbement et un maquillage chargé : les cils présentent une bonne épaisseur de maquillage.

### Exemple 2:

On prépare un mascara ayant la composition suivante :
- cire de son de riz 10 g
- cire de carnauba 8 g
- cire de Candelilla 1 g
- stéarate de triéthanolamine 9 g
- cire d'abeille 4 g
- hydrolysat de protéine de blé vendu sous la dénomination "Tritisol" par la société CRODA 0,4 g MA
- hydroxyethylcellulose 1,5 g
- pigments 5 g
- conservateurs qs
- eau qsp 100 g
- dimethicone copolyol vendu sous la dénomination " Q2-5520 " par la société Dow Corning 0,3 g

On obtiend ainsi un mascara conférant un bon recourbement aux cils après l'application. Le maquillage déposé sur les cils est épais et présente une bonne tenue dans le temps.

### Exemple 3:

On prépare un mascara ayant la composition suivante :
- cire de son de riz 8 g
- cire de carnauba 8 g
- cire de paraffine 2 g
- stéarate de triéthanolamine 9 g
- cire d'abeille 3 g
- copolymère de diglycol/cyclohexane dimethanol/ isophtalates/sulfoisophtalates vendu sous la dénomination " Eastman AQ-55S" par la société Eastman 1 g
- hydroxyéthylcellulose 1,2 g
- pigments 7 g
- conservateurs qs
- eau qsp 100 g

Après l'application de ce mascara sur les cils, on constate un bon recourbement des cils ainsi qu'un maquillage épais déposé sur les cils.

### Exemple 4 (invention) :

On a préparé un mascara ayant la composition suivante :
- cire de carnauba 20,1 g
- monostéarate de glycérol polyoxyéthyléné (30 OE) (TAGAT S de la société GOLDSCHMIDT) 6,71 g
- hydroxyéthylcellulose (Cellosize QP4400M de la société AMERCHOL° 1 g
- gomme arabique 1,5 g
- panthénol 1 g
- pigment 5 g
- soude qs pH 7
- conservateur qs
- eau qsp 100 g

### Exemple 5 : (comparatif)

On a préparé une microdispersion de cire, comme décrite dans la demande EP-A-557196, ayant la composition suivante :
- cire de carnauba 22,5 g
- Tagat S 7,5 g
- conservateur qs
- eau qsp 100 g

La microdispersion de cire présente une granulométrie moyenne de particules de cire de 283 nm.

Puis on a préparé, avec la microdispersion de cire, un mascara ayant la composition suivante :
- microdispersion de cire 89,5 g
- hydroxyéthylcellulose (Cellosize QP4400M de la société AMERCHOL° 1 g
- gomme arabique 1,5 g
- panthénol 1 g
- pigment 5 g
- soude qs pH 7
- conservateur qs
- eau qsp 100 g

On a maquillé des cils à l'aide des compositions des exemples 4 et 5 et comparé les résultats de maquillage obtenu : on a constaté que seuls les cils maquillés avec la composition de l'exemple 4 (invention) présentent un maquillage épais et recourbant.

### Exemples 6 à 9 :

On a préparé 3 mascaras selon l'invention (exemples 6 à 8) et un mascara ne faisant pas partie de l'invention (exemple 9) ayant la composition suivante :

| | **Exemple 6** **(invention)** | **Exemple 7** **(invention)** | **Exemple 8** **(invention)** | **Exemple 9** **(hors invention)** |
|---|---|---|---|---|
| cire de carnauba | 20.1 g | 8.375 g | 8.375 g | 9 g |
| cire d'abeille | - | - | - | 11.1 g |
| cire de son de riz | - | 8.375 g | 8.375 g | - |
| cire de candellila | - | 3.35 g | 3.35 g | - |
| acide stéarique | 4.65 g | 4.65 g | 4.65 g | 4.65 g |
| triethanolamine | 2.05 g | 2.05 g g | 2.05 g g | 2.05 g |
| eau | qsp 100 g | qsp 100 g | qsp 100 g | qsp 100 g |
| conservateur | 0.3 g | 0.3 g | 0.3 g | 0.3 g |
| oxyde de fer noir | 5 g | 5 g | 5 g | 5 g |
| hydroxyéthylcellulose (Cellosize QP4400M d'Amerchol) | 1 g | 1 g | 0.7 g | 1 g |
| gomme arabique | 1.5 g | 1.5 g | 1.5 g | 1.5 g |
| Hydrolysat de protéine de blé Tritisol | - | - | 0.3g MA | - |
| panthenol | 1 g | 1 g | 1 g | 1 g |
| TOTAL | 100 g g | 100 g | 100 g | 100 g |

On a maquillé des cils à l'aide des compositions des exemples 6 à 9 et comparé les résultats de maquillage obtenu : on a constaté que les cils maquillés avec les compositions des exemples 6 à 8 (invention) présentent un maquillage plus épais et plus recourbant que celui obtenu avec la composition de l'exemple 9 (ne faisant pas partie de l'invention).

## Revendications

1. Utilisation d'une composition de mascara pour recourber et épaissir les fibres kératiniques, notamment les cils, ladite composition comprenant :
- (i) une émulsion cires-dans-eau d'au moins une cire I, ou un mélange de cires 1, ayant une pénétrabilité à l'aiguille allant de 1 à 7,5 et un point de fusion allant de 70 °C à 110 °C, ladite cire I contenant au moins une cire (Ia, Ib) ayant un point de fusion allant de 77 °C à 110 °C, ladite cire I, ou leurs mélanges, étant présent en une teneur d'au moins 10 % en poids par rapport au poids total de la composition, la cire, ou leur mélange, étant sous forme de particules ayant une taille au moins supérieure ou égale à 1 µm,
- (ii) au moins 0,1 % en poids, par rapport au poids total de la composition, d'un système polymérique contenant un polymère filmogène, ledit système polymérique étant apte à former un film produisant à une concentration de 7 % dans l'eau, une rétraction du stratum corneum isolé de plus de 1 % à 30°C sous une humidité relative de 40 %.

2. Utilisation selon la revendication 1, caractérisée par le fait que la cire (I) comprend au moins une cire (la) ayant un point de fusion supérieur ou égal à 77 °C et inférieur à 83 °C.

3. Utilisation selon la revendication 2, caractérisée par le fait que la cire (la) est la cire de son de riz.

4. Utilisation selon l'une quelconque des revendications précédentes, caractérisée par le fait que la cire (I) comprend au moins une cire (Ib) ayant un point de fusion allant de 83 °C à 110 °C.

5. Utilisation selon l'une quelconque des revendications précédentes, caractérisée par le fait que la cire (Ib) est choisie dans le groupe formé par la cire d'Ouricuri, la cire de carnauba, les cires de Montan.

6. Utilisation selon la revendication 4 ou 5, caractérisée par le fait que la cire (lb) est la cire de carnauba.

7. Utilisation selon l'une quelconque des revendications précédentes, caractérisée par le fait que la composition comprend un mélange d'au moins une cire (la) ayant un point de fusion supérieur ou égal à 77 °C et inférieur à 83 °C et au moins une cire (Ib) ayant un point de fusion allant de 83 °C à 110 °C.

8. Utilisation selon la revendication 7, caractérisée par le fait que ledit mélange de cires (la, Ib) comprend de 35 % à 65 % en poids de cire (la), par rapport au poids' total dudit mélange de cires I, et de 65 % à 35 % en poids de cire (lb).

9. Utilisation selon l'une quelconque des revendications précédentes, caractérisée par le fait que la composition comprend au moins une cire (Ic) ayant un point de fusion supérieur ou égal à 70 °C et inférieur à 77 °C.

10. Utilisation selon la revendication 9, caractérisée par le fait que ledit mélange de cire (la, Ib, Ic) comprend de 5 % à 20 % en poids de cire (Ic) par rapport au poids total dudit mélange de cires (la, Ib, Ic).

11. Utilisation selon la revendication 9 ou 10, caractérisée par le fait que la cire (lc) est la cire de Candelilla.

12. Utilisation selon l'une quelconque des revendications précédentes, caractérisée par le fait que la cire (I), ou leurs mélanges, est présente en une quantité d'au moins 15 % en poids par rapport au poids total de la composition.

13. Utilisation selon l'une quelconque des revendications précédentes, caractérisée par le fait que la taille des particules de cires est supérieure ou égale à 1,5 µm.

14. Utilisation selon l'une quelconque des revendications précédentes, caractérisée par le fait que le polymère filmogène est choisi dans le groupe formé par les polymères d'origine végétale, les polymères d'origine animale, les polymères radicalaires, les polycondensats.

15. Utilisation selon l'une quelconque des revendications précédentes, caractérisée par le fait que le polymère filmogène est un polymère hydrosoluble ou hydrodispersible.

16. Utilisation selon l'une quelconque des revendications précédentes, caractérisée par le fait que le polymère filmogène est choisi dans le groupe formé par les protéines, les hydrolysats de protéine, la chitine et ses dérivés, les polyester-polyuréthanes, les sulfopolyesters.

17. Utilisation selon la revendication 15, caractérisée par le fait que le polymère filmogène est un hydrolysat de protéine de blé.

18. Utilisation selon l'une quelconque des revendications précédentes, caractérisée par le fait que la composition comprend au moins une cire Il ayant un point de fusion supérieur ou égal à 50 °C et inférieur à 70 °C et une pénétrabilité à l'aiguille supérieure à 7,5.

19. Utilisation selon la revendication 18, caractérisée par le fait que le rapport pondéral (poids de cires I /poids de cire II) va de 2 à 5.

20. Utilisation selon l'une quelconque des revendications précédentes, caractérisée par le fait qu'elle comprend un agent épaississant.

21. Utilisation selon l'une quelconque des revendications précédentes, caractérisée par le fait qu'elle comprend au moins un tensioactif siliconé ayant un HLB allant de 8 à 16.

22. Utilisation selon la revendication 21, caractérisée par le fait que le tensioactif siliconé est un diméthicone copolyol.

23. Composition de mascara comprenant :
(i) une émulsion cires-dans-eau d'au moins un mélange de cires I ayant une pénétrabilité à l'aiguille allant de 1 à 7,5, et un point de fusion allant de 70 °C à 110 °C, ledit mélange de cires I contenant au moins une première cire (la) ayant un point de fusion supérieur ou égal à 77 °C et inférieur à 83 °C, une deuxième cire (Ib) ayant un point de fusion allant de 83 °C à 110 °C, et une troisième cire (Ic) ayant un point de fusion supérieur ou égal à 70 °C et inférieur à 77 °C, ledit mélange de cires I étant présent en une teneur d'au moins 10 % en poids par rapport au poids total de la composition, ledit mélange de cires I étant sous forme de particules ayant une taille au moins supérieure ou égale à 1 µm,
(ii) au moins 0,1 % en poids, par rapport au poids total de la composition, d'un système polymérique contenant un polymère filmogène, ledit système polymérique étant apte à former un film produisant à une concentration de 7 % dans l'eau, une rétraction du stratum comeum isolé de plus de 1 % à 30°C sous une humidité relative de 40 %.

24. Composition selon la revendication 23, caractérisée par le fait que la première cire (la) est la cire de son de riz.

25. Composition selon la revendication 23 ou 24, caractérisée par le fait que la deuxième cire (Ib) est choisie dans le groupe formé par la cire de Carnauba, la cire d'Ouricuri, les cires de Montan.

26. Composition selon l'une quelconque des revendications 23 à 25, caractérisée par le fait que la deuxième cire (Ib) est la cire de carnauba.

27. Composition cosmétique selon l'une quelconque des revendications 23 à 26, caractérisée par le fait que la troisième cire (Ic) est la cire de Candelilla.

28. Composition selon l'une quelconque des revendications 23 à 27, caractérisée par le fait qu'elle comprend au moins 15 % en poids, par rapport au poids total de la composition, dudit mélange de cires I.

29. Composition selon l'une quelconque des revendications 23 à 28, caractérisée par le fait que les première, deuxième et troisième cires (la, Ib, Ic) sont présentes selon un rapport pondéral (poids/poids total de cire I) allant respectivement de :
- cire (la) : 0,35 à 0,5,
- cire (lb) : 0,35 à 0,5,
- cire (lc) : 0,05 à 0,2.

30. Composition selon l'une quelconque des revendications 23 à 29, caractérisée par le fait que la taille des particules de cires est supérieure ou égale à 1,5 µm.

31. Composition selon l'une quelconque des revendications 23 à 30, caractérisée par le fait que le polymère filmogène est choisi dans le groupe formé par les polymères d'origine végétale, les polymères d'origine animale, les polymères radicalaires, les polycondensats.

32. Composition selon l'une quelconque des revendications 23 à 31, caractérisée par le fait que le polymère filmogène est un polymère hydrosoluble ou hydrodispersible.

33. Composition selon l'une quelconque des revendications 23 à 32, caractérisée par le fait que le polymère filmogène est choisi dans le groupe formé par les protéines, les hydrolysats de protéine, la chitine et ses dérivés, les polyester-polyuréthanes, les sulfopolyesters.

34. Composition selon l'une quelconque des revendications 23 à 33, caractérisée par le fait que le polymère filmogène est un hydrolysat de protéine de blé.

35. Composition selon l'une quelconque des revendications 23 à 34, caractérisée par le fait qu'elle comprend au moins une cire Il ayant un point de fusion supérieur ou égal à 50 °C et inférieur à 70 °C et une pénétrabilité à l'aiguille supérieure à 7,5.

36. Composition selon la revendication 35, caractérisée par le fait que le rapport pondéral (poids de cires I /poids de cire II) va de 2 à 5.

37. Composition selon l'une quelconque des revendications 23 à 36, caractérisée par le fait qu'elle comprend au moins un agent épaississant.

38. Composition selon l'une quelconque des revendications 23 à 37, caractérisée par le fait qu'elle comprend au moins un tensioactif siliconé ayant un HLB allant de 8 à 16.

39. Composition selon la revendication 38, caractérisée par le fait que le tensioactif siliconé est un diméthicone copolyol.

40. Produit de mascara comprenant un réservoir, contenant une composition de mascara, et système d'application de ladite composition sur les fibres kératiniques, notamment les cils, caractérisé par le fait que la composition est une composition selon l'une quelconque des revendications 23 à 39.

41. Procédé de maquillage des fibres kératiniques, notamment des cils, caractérisé par le fait que l'on applique sur les fibres kératiniques une composition selon l'une quelconque des revendications 23 à 39.

## Claims

1. Use of a mascara composition for curling and thickening keratin fibres, in particular the eyelashes, the said composition comprising:
- (i) a waxes-in-water emulsion of at least one wax I, or a mixture of waxes I, having a needle penetration ranging from 1 to 7.5 and a melting point ranging from 70°C to 110°C, the said wax I containing at least one wax (Ia, Ib) having a melting point ranging from 77°C to 110°C, the said wax I, or their mixtures, being present in a content of at least 10% by weight relative to the total weight of the composition, the wax, or their mixture, being in the form of particles greater than or equal to at least 1 µm in size,
- (ii) at least 0.1% by weight, relative to the total weight of the composition, of a polymer system containing a film-forming polymer, the said polymer system being capable of forming a film which produces, at a concentration of 7% in water, a greater than 1% retraction of isolated stratum corneum at 30°C and under a relative humidity of 40%.

2. Use according to Claim 1, characterized in that the wax (I) comprises at least one wax (Ia) having a melting point of greater than or equal to 77°C and less than 83°C.

3. Use according to Claim 2, characterized in that the wax (Ia) is rice bran wax.

4. Use according to any one of the preceding claims, characterized in that the wax (I) comprises at least one wax (Ib) having a melting point ranging from 83°C to 110°C.

5. Use according to any one of the preceding claims, characterized in that the wax (Ib) is chosen from the group formed by ouricurry wax, carnauba wax and montan waxes.

6. Use according to Claim 4 or 5, characterized in that the wax (Ib) is carnauba wax.

7. Use according to any one of the preceding claims, characterized in that the composition comprises a mixture of at least one wax (Ia) having a melting point of greater than or equal to 77°C and less than 83°C and at least one wax (Ib) having a melting point ranging from 83°C to 110°C.

8. Use according to Claim 7, characterized in that the said mixture of waxes (Ia, Ib) comprises from 35% to 65% by weight of wax (Ia), relative to the total weight of the said mixture of waxes I, and from 65% to 35% by weight of wax (Ib).

9. Use according to any one of the preceding claims, characterized in that the composition comprises at least one wax (Ic) having a melting point of greater than or equal to 70°C and less than 77°C.

10. Use according to Claim 9, characterized in that the said mixture of wax (Ia, Ib, Ic) comprises from 5% to 20% by weight of wax (Ic) relative to the total weight of the said mixture of waxes (Ia, Ib, Ic).

11. Use according to Claim 9 or 10, characterized in that the wax (Ic) is candelilla wax.

12. Use according to any one of the preceding claims, characterized in that the wax (I), or their mixtures, is/are present in an amount of at least 15% by weight relative to the total weight of the composition.

13. Use according to any one of the preceding claims, characterized in that the wax particles are greater than or equal to 1.5 µm in size.

14. Use according to any one of the preceding claims, characterized in that the film-forming polymer is chosen from the group formed by polymers of plant origin, polymers of animal origin, radical polymers and polycondensates.

15. Use according to any one of the preceding claims, characterized in that the film-forming polymer is a water-soluble or water-dispersible polymer.

16. Use according to any one of the preceding claims, characterized in that the film-forming polymer is chosen from the group formed by proteins, protein hydrolysates, chitin and its derivatives, polyester-polyurethanes and sulphopolyesters.

17. Use according to Claim 15, characterized in that the film-forming polymer is a wheat protein hydrolysate.

18. Use according to any one of the preceding claims, characterized in that the composition comprises at least one wax II having a melting point of greater than or equal to 50°C and less than 70°C and a needle penetration of greater than 7.5.

19. Use according to Claim 18, characterized in that the weight ratio (weight of waxes I/weight of wax II) ranges from 2 to 5.

20. Use according to any one of the preceding claims, characterized in that the composition comprises a thickener.

21. Use according to any one of the preceding claims, characterized in that the composition comprises at least one silicone surfactant having an HLB ranging from 8 to 16.

22. Use according to Claim 21, characterized in that the silicone surfactant is a dimethicone copolyol.

23. Mascara composition comprising:
- (i) a waxes-in-water emulsion of at least one mixture of waxes I having a needle penetration ranging from 1 to 7.5 and a melting point ranging from 70°C to 110°C, the said mixture of waxes I containing at least a first wax (Ia) having a melting point greater than or equal to 77°C and less than 83°C, a second wax (Ib) having a melting point ranging from 83°C to 110°C, and a third wax (Ic) having a melting point greater than or equal to 70°C and less than 77°C, the said mixture of waxes I being present in a content of at least 10% by weight relative to the total weight of the composition, the said mixture of waxes I being in the form of particles greater than or equal to at least 1 µm in size,
- (ii) at least 0.1% by weight, relative to the total weight of the composition, of a polymer system containing a film-forming polymer, the said polymer system being capable of forming a film which produces, at a concentration of 7% in water, a greater than 1% retraction of isolated stratum corneum at 30°C and under a relative humidity of 40%.

24. Composition according to Claim 23, characterized in that the first wax (Ia) is rice bran wax.

25. Composition according to Claim 23 or 24, characterized in that the second wax (Ib) is chosen from the group formed by carnauba wax, ouricurry wax and montan waxes.

26. Composition according to any one of Claims 23 to 25, characterized in that the second wax (Ib) is carnauba wax.

27. Cosmetic composition according to any one of Claims 23 to 26, characterized in that the third wax (Ic) is candelilla wax.

28. Composition according to any one of Claims 23 to 27, characterized in that it comprises at least 15% by weight, relative to the total weight of the composition, of the said mixture of waxes I.

29. Composition according to any one of Claims 23 to 28, characterized in that the first, second and third waxes (Ia, Ib, Ic) are present in a weight ratio (weight/total weight of wax I) ranging, respectively, from:
- wax (Ia) : 0.35 to 0.5,
- wax (Ib) : 0.35 to 0.5,
- wax (Ic) : 0.05 to 0.2.

30. Composition according to any one of Claims 23 to 29, characterized in that the wax particles are greater than or equal to 1.5 µm in size.

31. Composition according to any one of Claims 23 to 30, characterized in that the film-forming polymer is chosen from the group formed by polymers of plant origin, polymers of animal origin, radical polymers and polycondensates.

32. Composition according to any one of Claims 23 to 31, characterized in that the film-forming polymer is a water-soluble or water-dispersible polymer.

33. Composition according to any one of Claims 23 to 32, characterized in that the film-forming polymer is chosen from the group formed by proteins, protein hydrolysates, chitin and its derivatives, polyester-polyurethanes and sulphopolyesters.

34. Composition according to any one of Claims 23 to 33, characterized in that the film-forming polymer is a wheat protein hydrolysate.

35. Composition according to any one .of Claims 23 to 34, characterized in that it comprises at least one wax II having a melting point of greater than or equal to 50°C and less than 70°C and a needle penetration of greater than 7.5.

36. Composition according to Claim 35, characterized in that the weight ratio (weight of waxes I/weight of wax II) ranges from 2 to 5.

37. Composition according to any one of Claims 23 to 36, characterized in that it comprises at least one thickener.

38. Composition according to any one of Claims 23 to 37, characterized in that it comprises at least one silicone surfactant having an HLB ranging from 8 to 16.

39. Composition according to Claim 38, characterized in that the silicone surfactant is a dimethicone copolyol.

40. Mascara product comprising a reservoir, containing a mascara composition, and a system for applying the said composition to keratin fibres, in particular the eyelashes, characterized in that the composition is a composition according to any one of Claims 23 to 39.

41. Process for making up keratin fibres, in particular the eyelashes, characterized in that a composition according to any one of Claims 23 to 39 is applied to the keratin fibres.

## Patentansprüche

1. Verwendung einer Mascara-Zusammensetzung, um Keratinfasern und insbesondere Wimpern eine geschwungene Form zu geben und sie dichter zu machen, wobei die Zusammensetzung enthält:
- (i) eine Wachs-in-Wasser-Emulsion aus mindestens einem Wachs I oder einem Gemisch von Wachsen I mit einem Index der Nadelpenetration von 1 bis 7,5 und einem Schmelzpunkt von 70 bis 110 °C, wobei das Wachs I mindestens ein Wachs (Ia, Ib) mit einem Schmelzpunkt von 77 bis 110 °C enthält, wobei das Wachs I oder die Gemische von Wachsen I in einem Mengenanteil von mindestens 10 Gew.-%, bezogen auf das Gesamtgewicht der Zusammensetzung, vorliegen und wobei das Wachs oder das Gemisch von Wachsen in Form von Partikeln mit einer Größe von mindestens größer oder gleich 1 µm vorliegt, und
- (ii) bezogen auf das Gesamtgewicht der Zusammensetzung mindestens 0,1 Gew.-% eines Polymersystems, das ein filmbildendes Polymer enthält, wobei das Polymersystem befähigt ist, einen Film zu bilden, der bei einer Konzentration von 7 % in Wasser bei 30 °C und einer relativen Feuchte von 40 % eine Dehnung von isoliertem Stratum corneum von mehr als 1 % hervorruft.

2. Verwendung nach Anspruch 1, dadurch gekennzeichnet, dass das Wachs (I) mindestens ein Wachs (Ia) mit einem Schmelzpunkt von größer oder gleich 77 °C und kleiner 83 °C umfasst.

3. Verwendung nach Anspruch 2, dadurch gekennzeichnet, dass das Wachs (Ia) Reiskleiewachs ist.

4. Verwendung nach einem der vorhergehenden Ansprüche, dadurch gekennzeichnet, dass das Wachs (I) mindestens ein Wachs (Ib) mit einem Schmelzpunkt von 83 bis 110 °C umfasst.

5. Verwendung nach einem der vorhergehenden Ansprüche, dadurch gekennzeichnet, dass das Wachs (Ib) unter Ouricouriwachs, Carnaubawachs und Montanwachsen ausgewählt ist.

6. Verwendung nach Anspruch 4 oder 5, dadurch gekennzeichnet, dass das Wachs (Ib) Carnaubawachs ist.

7. Verwendung nach einem der vorhergehenden Ansprüche, dadurch gekennzeichnet, dass die Zusammensetzung ein Gemisch von mindestens einem Wachs (Ia) mit einem Schmelzpunkt von größer oder gleich 77 °C und kleiner 83 °C und mindestens ein Wachs (Ib) mit einem Schmelzpunkt von 83 bis 110 °C enthält.

8. Verwendung nach Anspruch 7, dadurch gekennzeichnet, dass das Gemisch von Wachsen (Ia, Ib) 35 bis 65 Gew.-% Wachs (Ia) und 65 bis 35 Gew.-% Wachs (Ib), bezogen auf das Gesamtgewicht des Gemisches von Wachsen I, enthält.

9. Verwendung nach einem der vorhergehenden Ansprüche, dadurch gekennzeichnet, dass die Zusammensetzung mindestens ein Wachs (Ic) mit einem Schmelzpunkt von größer oder gleich 70 °C und kleiner 77 °C enthält.

10. Verwendung nach Anspruch 9, dadurch gekennzeichnet, dass das Gemisch von Wachsen (Ia, Ib, Ic) 5 bis 20 Gew.-% Wachs (Ic), bezogen auf das Gesamtgewicht des Gemisches von Wachsen (Ia, Ib, Ic), enthält.

11. Verwendung nach Anspruch 9 oder 10, dadurch gekennzeichnet, dass das Wachs (Ic) Candelillawachs ist.

12. Verwendung nach einem der vorhergehenden Ansprüche, dadurch gekennzeichnet, dass das Wachs I oder die Gemische von Wachsen I in einem Mengenanteil von mindestens 15 Gew.-%, bezogen auf das Gesamtgewicht der Zusammensetzung, vorliegen.

13. Verwendung nach einem der vorhergehenden Ansprüche, dadurch gekennzeichnet, dass die Größe der Wachspartikel größer oder gleich 1,5 µm ist.

14. Verwendung nach einem der vorhergehenden Ansprüche, dadurch gekennzeichnet, dass das filmbildende Polymer unter den Polymeren pflanzlichen Ursprungs, den Polymeren tierischen Ursprungs, durch radikalische Polymerisation hergestellten Polymeren und Polykondensaten ausgewählt ist.

15. Verwendung nach einem der vorhergehenden Ansprüche, dadurch gekennzeichnet, dass das filmbildende Polymer ein wasserlösliches oder in Wasser dispergierbares Polymer ist.

16. Verwendung nach einem der vorhergehenden Ansprüche, dadurch gekennzeichnet, dass das filmbildende Polymer unter den Proteinen, Proteinhydrolysaten, Chitin und seinen Derivaten, Polyester-Polyurethanen und Sulfopolyestern ausgewählt ist.

17. Verwendung nach Anspruch 15, dadurch gekennzeichnet, dass das filmbildende Polymer ein Weizenproteinhydrolysat ist.

18. Verwendung nach einem der vorhergehenden Ansprüche, dadurch gekennzeichnet, dass die Zusammensetzung mindestens ein Wachs II mit einem Schmelzpunkt von größer oder gleich 50 °C und kleiner 70 °C und einem Index der Nadelpenetration von größer 7,5 enthält.

19. Verwendung nach Anspruch 18, dadurch gekennzeichnet, dass das Gewichtsverhältnis (Gewicht von Wachsen I/Gewicht von Wachs II) im Bereich von 2 bis 5 liegt.

20. Verwendung nach einem der vorhergehenden Ansprüche, dadurch gekennzeichnet, dass sie ein Verdickungsmittel aufweist.

21. Verwendung nach einem der vorhergehenden Ansprüche, dadurch gekennzeichnet, dass sie mindestens einen siliconhaltigen grenzflächenaktiven Stoff mit einem HLB-Wert von 8 bis 16 aufweist.

22. Verwendung nach Anspruch 21, dadurch gekennzeichnet, dass der siliconhaltige grenzflächenaktive Stoff ein Dimeticoncopolyol ist.

23. Mascara-Zusammensetzung, die enthält:
- (i) eine Wachs-in-Wasser-Emulsion aus mindestens einem Gemisch von Wachsen I mit einem Index der Nadelpenetration von 1 bis 7,5 und einem Schmelzpunkt von 70 bis 110 °C, wobei das Gemisch von Wachsen I mindestens ein erstes Wachs (Ia) mit einem Schmelzpunkt von größer oder gleich 77 °C und kleiner 83 °C, ein zweites Wachs (Ib) mit einem Schmelzpunkt von 83 bis 110 °C und ein drittes Wachs (Ic) mit einem Schmelzpunkt von größer oder gleich 70 °C und kleiner 77°C enthält, wobei das Gemisch von Wachsen I in einem Mengenanteil von mindestens 10 Gew.-%, bezogen auf das Gesamtgewicht der Zusammensetzung, vorliegt und wobei das Gemisch von Wachsen I in Form von Partikeln mit einer Größe von mindestens größer oder gleich 1 µm vorliegt, und
- (ii) bezogen auf das Gesamtgewicht der Zusammensetzung mindestens 0,1 Gew.-% eines Polymersystems, das ein filmbildendes Polymer enthält, wobei das Polymersystem befähigt ist, einen Film zu bilden, der bei einer Konzentration von 7 % in Wasser bei 30 °C und einer relativen Feuchte von 40 % eine Dehnung von isoliertem Stratum corneum von mehr als 1 % hervorruft.

24. Zusammensetzung nach Anspruch 23, dadurch gekennzeichnet, dass das erste Wachs (Ia) Reiskleiewachs ist.

25. Zusammensetzung nach Anspruch 23 oder 24, dadurch gekennzeichnet, dass das zweite Wachs (Ib) unter Carnaubawachs, Ouricouriwachs und Montanwachsen ausgewählt ist.

26. Zusammensetzung nach einem der Ansprüche 23 bis 25, dadurch gekennzeichnet, dass das zweite Wachs (Ib) Carnaubawachs ist.

27. Kosmetische Zusammensetzung nach einem der Ansprüche 23 bis 26, dadurch gekennzeichnet, dass das dritte Wachs (Ic) Candelillawachs ist.

28. Zusammensetzung nach einem der Ansprüche 23 bis 27, dadurch gekennzeichnet, dass sie mindestens 15 Gew.-% des Gemisches von Wachsen I, bezogen auf das Gesamtgewicht der Zusammensetzung, enthält.

29. Zusammensetzung nach einem der Ansprüche 23 bis 28, dadurch gekennzeichnet, dass das erste Wachs, das zweite Wachs und das dritte Wachs (Ia, Ib, Ic) in den folgenden Gewichtsverhältnissen (Gewicht/Gesamtgewicht Wachs I) vorliegen:
- Wachs (Ia): 0,35 bis 0,5,
- Wachs (Ib): 0,35 bis 0,5 und
- Wachs (Ic): 0,05 bis 0,2.

30. Zusammensetzung nach einem der Ansprüche 23 bis 29, dadurch gekennzeichnet, dass die Größe der Wachspartikel größer oder gleich 1,5 µm ist.

31. Zusammensetzung nach einem der Ansprüche 23 bis 30, dadurch gekennzeichnet, dass das filmbildende Polymer unter den Polymeren pflanzlichen Ursprungs, den Polymeren tierischen Ursprungs, durch radikalische Polymerisation hergestellten Polymeren und Polykondensaten ausgewählt ist.

32. Zusammensetzung nach einem der Ansprüche 23 bis 31, dadurch gekennzeichnet, dass das filmbildende Polymer ein wasserlösliches oder in Wasser dispergierbares Polymer ist.

33. Zusammensetzung nach einem der Ansprüche 23 bis 32, dadurch gekennzeichnet, dass das filmbildende Polymer unter den Proteinen, Proteinhydrolysaten, Chitin und seinen Derivaten, Polyester-Polyurethanen und Sulfopolyestem ausgewählt ist.

34. Zusammensetzung nach einem der Ansprüche 23 bis 33, dadurch gekennzeichnet, dass das filmbildende Polymer ein Weizenproteinhydrolysat ist.

35. Zusammensetzung nach einem der Ansprüche 23 bis 34, dadurch gekennzeichnet, dass sie mindestens ein Wachs II mit einem Schmelzpunkt von größer oder gleich 50 °C und kleiner 70 °C und einem Index der Nadelpenetration von größer 7,5 enthält.

36. Zusammensetzung nach Anspruch 35, dadurch gekennzeichnet, dass das Gewichtsverhältnis (Gewicht von Wachsen I/Gewicht von Wachs II) im Bereich von 2 bis 5 liegt.

37. Zusammensetzung nach einem der Ansprüche 23 bis 36, dadurch gekennzeichnet, dass sie mindestens ein Verdickungsmittel enthält.

38. Zusammensetzung nach einem der Ansprüche 23 bis 37, dadurch gekennzeichnet, dass sie mindestens einen siliconhaltigen grenzflächenaktiven Stoff mit einem HLB-Wert von 8 bis 16 enthält.

39. Zusammensetzung nach Anspruch 38, dadurch gekennzeichnet, dass der siliconhaltige grenzflächenaktive Stoff ein Dimeticoncopolyol ist.

40. Mascara-Produkt, das einen Behälter, der eine Mascara-Zusammensetzung enthält, und ein System zum Auftragen dieser Zusammensetzung auf die Keratinfasern und insbesondere die Wimpern umfasst, dadurch gekennzeichnet, dass die Zusammensetzung eine Zusammensetzung nach einem der Ansprüche 23 bis 39 ist.

41. Verfahren zum Schminken von Keratinfasern und insbesondere der Wimpern, dadurch gekennzeichnet, dass eine Zusammensetzung nach einem der Ansprüche 23 bis 39 auf die Keratinfasern aufgebracht wird.
